(19) Europäisches Patentamt
European Patent Office
Office européen des brevets

(11) Veröffentlichungsnummer : **0 472 093 B1**

(12) **EUROPÄISCHE PATENTSCHRIFT**

(45) Veröffentlichungstag der Patentschrift : **03.11.93 Patentblatt 93/44**

(51) Int. Cl.[5] : **C07C 279/12,** C07C 279/16, C07D 233/48, C07D 239/14, C07D 245/02, C07D 247/02, A01N 47/44, A01N 43/48

(21) Anmeldenummer : **91113556.4**

(22) Anmeldetag : **13.08.91**

(54) **Bis-Guanidine und diese enthaltende Fungizide.**

(30) Priorität : **22.08.90 DE 4026473**

(43) Veröffentlichungstag der Anmeldung : **26.02.92 Patentblatt 92/09**

(45) Bekanntmachung des Hinweises auf die Patenterteilung : **03.11.93 Patentblatt 93/44**

(84) Benannte Vertragsstaaten : **AT BE CH DE DK ES FR GB GR IT LI NL SE**

(56) Entgegenhaltungen :
**EP-A- 0 338 430**
**EP-A- 0 406 699**
**FR-A- 1 271 180**
**GB-A- 935 614**
**GB-A- 1 294 443**
**US-A- 3 468 898**
**US-A- 3 905 992**
**CHEMICAL ABSTRACTS, vol. 85, no. 11, 13. September 1976, Columbus, Ohio, US; abstract no. 72190Q, H.TOMOMATSU ET.AL.: 'The anti-tumor effect of polyguanidino compounds.VII.Effect of bifunctional methylguanidine derivatives on Ehrlich ascites tumor cells.' Seite 34 ;Spalte 2 ;**

(56) Entgegenhaltungen :
**CHEMICAL ABSTRACTS, vol. 98, no. 1, 3. Januar 1983, Columbus, Ohio, US; abstract no. 179P, A.V.BOGATSKII ET.AL.: 'Effect of polymethylene-and poly(oxyethylene)bis(2-amino-1 ,3-diazepine)iodides on cellular and model membranes.' Seite 16 ;Spalte 2 ;**
**CHEMICAL ABSTRACTS, vol. 98, no. 25, 20. Juni 1983, Columbus, Ohio, US; abstract no. 215574Z, A.V.BOGATSKII ET.AL.: 'Macroheterocycles.Part II.Synthesis and bacteriostatic activity of 2,2-(polymethylenediamino)-and 2,2- (polyhydroxy ethylenediamino-)bis(4,5,6,7-tetrahydro-1,3-diaze pinium iodides).' Seite 559 ;Spalte 2 ; EP 91113556030**

(73) Patentinhaber : **BASF Aktiengesellschaft**
**Carl-Bosch-Strasse 38**
**D-67063 Ludwigshafen (DE)**

(72) Erfinder : **Mueller, Thomas, Dr.**
**Bergstrasse 19**
**W-6717 Hessheim (DE)**
Erfinder : **Zipplies, Matthias, Dr.**
**Kastanienweg 1**
**W-6945 Hirschberg (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

**EP 0 472 093 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft neue Bis-Guanidine mit fungizider Wirkung und diese enthaltende Fungizide.

Aus GB 1 114 155 ist die Verbindung

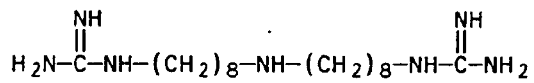

und ihre fungizide Wirkung bekannt.

Weiterhin ist die fungizide Wirkung von α,ω-Bis-(3,4-dichlorbenzylguanidino)alkanen bekannt (A. F. McKay et al., J. Med. Chem. 6 (1963) 587).

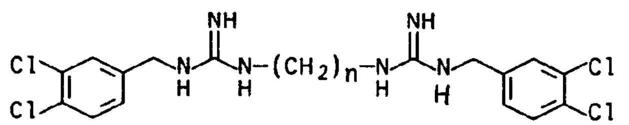

n = 10, 11, 12

Weitere Guanidine sind bekannt aus US 3 468 898, DE 39 22 232.2, EP 338 430, GB 935 614 und EP 406 699.

Die Wirkung dieser Verbindungen ist jedoch insbesondere bei niedrigen Aufwandmengen und -konzentrationen nicht immer befriedigend.

Es wurde nun gefunden, daß Bis-Guanidin-Derivate der allgemeinen Formel I,

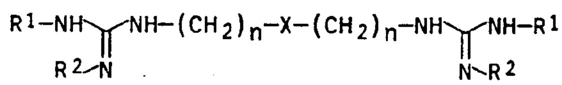

I

in der

$R^1$ und $R^2$ unabhängig voneinander Wasserstoff, $C_3$-$C_{10}$-Al-kenyl, $C_3$-$C_{10}$-Alkinyl, $C_1$-$C_8$-Alkoxy-$C_2$-$C_8$-alkyl, $C_3$-$C_{10}$-Halogenalkenyl; oder eine Cycloalkylgruppe mit 5 bis 12 Kohlenstoffatomen im Ring, die bis zu drei der folgenden Substituenten
Hydroxyl, $C_1$-$C_{10}$-Alkyl, $C_1$-$C_{10}$-Alkoxy, $C_1$-$C_{10}$-Halogenalkyl und $C_1$-$C_8$-Alkoxy-$C_1$-$C_8$-alkyl tragen kann;
oder eine Benzylgruppe, die durch bis zu drei $C_1$-$C_{10}$-Alkyl- oder $C_1$-$C_{10}$-Alkoxy-Gruppen substituiert sein kann, wobei der Substituent noch eine Hydroxyl- oder $C_1$-$C_6$-Alkoxygruppe tragen kann; oder die Benzylgruppe durch eine $C_1$-$C_{10}$-Halogenalkyl-Gruppe substituiert ist,
mit der Maßgabe, daß nur einer der beiden Substituenten $R^1$ oder $R^2$ Wasserstoff sein kann; oder

$R^1$ und $R^2$ zusammen mit den Atomen, deren Substituenten sie sind, einen 5- bis 11-gliedrigen heterocyclischen Ring bedeuten, der durch C1-$C_{10}$-Alkyl oder $C_1$-$C_8$-Alkoxy-$C_1$-$C_8$-alkyl ein- bis dreifach substituiert sein kann;

X $CH_2$, O, eine C-C-Einfachbindung, NH, N-($C_1$-$C_{10}$-Alkyl) oder N-Benzyl, wobei der Phenylring des Benzylrestes einfach bis dreifach durch $C_1$-$C_{10}$-Alkyl, $C_1$-$C_{10}$-Alkoxy, $C_1$-$C_{10}$-Halogenalkyl oder Halogen substituiert sein kann,

n 5 bis 8 bedeuten,

sowie ihre pflanzenverträglichen Säureadditionssalze und ihre Metallkomplexe eine ausgezeichnete fungizide Wirkung gegen phytopathogene Pilze besitzen.

Wenn $R^1$ oder $R^2$ Wasserstoff bedeuten, können die Verbindungen in tautomeren Formen vorliegen, die von der Erfindung umfaßt werden.

Im Hinblick auf ihre fungizide Wirkung werden diejenigen Verbindungen bevorzugt, in denen die Substituenten folgende Bedeutung haben:

$R^1$, $R^2$

Wasserstoff, mit der Maßgabe, daß mindestens einer der Substituenten $R^1$, $R^2$ verschieden von Wasserstoff ist;

$C_3$-$C_{10}$-Alkenyl oder $C_3$-$C_{10}$-Alkinyl, insbesondere die Allyl-, Dimethylallyl- oder die But-2-inyl-gruppe;

$C_1$-$C_8$-Alkoxy-$C_2$-$C_8$-alkyl, insbesondere die Methoxy-ethyl-, tert.-Butoxy-ethyl oder die Methoxy-propylgruppe;
$C_3$-$C_{10}$-Halogenalkenyl, insbesondere die 3-Brom-prop-2-enyl-, 2-Brom-prop-2-enyl- oder die 3-Chlor-prop-2-enyl-gruppe;
Cyclopentyl, Cyclohexyl, Cycloheptyl, Cyclooctyl und Cyclododecyl, wobei diese Gruppen bis zu drei der folgenden Reste tragen können:

Hydroxyl;

$C_1$-$C_{10}$-Alkylgruppen, insbesondere die Methyl-, Ethyl-, Propyl-, Isopropyl-, Butyl-, Isobutyl-, sek.-Butyl-, tert.-Butyl-, 1,1-Dimethylpropyl oder die 2,4,4-Trimethyl-pent-2-yl-gruppe;

$C_1$-$C_{10}$-Alkoxygruppen, insbesondere die Methoxy-, Ethoxy-, Isopropoxy-, n-Butoxy-, tert.-Butoxy- oder die Octyloxygruppe;

$C_1$-$C_4$-Halogenalkylgruppen, insbesondere die Trifluormethylgruppe oder die Pentafluorethylgruppe;

$C_1$-$C_8$-Alkoxy-$C_1$-$C_8$-alkylgruppen, insbesondere die Methoxy-methyl-, Ethoxy-methyl-, Methoxy-ethyl-, tert.-Butoxy-methyl- oder die 1-Methoxy-1-methyl-ethylgruppe; oder

Benzyl, wobei diese Gruppe bis zu drei der folgenden Substituenten tragen kann:

die $C_1$-$C_{10}$-Alkylgruppe, insbesondere die Methyl-, Ethyl-, Propyl-, Isopropyl-, Butyl-, Isobutyl-, sek.-Butyl-, tert.-Butyl-, 1,1-Dimethylethyl-, 1,1-Dimethylpropyl-, 2,3-Dimethylpropyl-, 1,1,2-Trimethylpropyl-, 2-Hydroxy-prop-2-yl- oder die 2-Methoxy-prop-2-yl-gruppe;

die $C_1$-$C_{10}$-Alkoxygruppe, insbesondere die Methoxy-, Ethoxy-, Isopropoxy-, n-Butoxy-, tert.-Butoxy- oder die Octyloxygruppe;

die $C_1$-$C_{10}$-Halogenalkylgruppe, insbesondere die Trifluormethyl- oder die Pentafluorethylgruppe;

besonders bevorzugt unter den Resten $R^1$ und $R^2$ werden Wasserstoff, mit der Maßgabe, daß mindestens einer der Substituenten $R^1$, $R^2$ verschieden von Wasserstoff ist, Cyclopentyl, Cyclohexyl, Cycloheptyl, 2-Methylcyclohexyl, 3-Methylcyclohexyl, 3-Trifluormethylcyclohexyl, 3,3-Dimethylcyclohexyl, 4-tert.-Butylcyclohexyl, 4-Methylbenzyl, 4-Ethylbenzyl, 4-Isopropylbenzyl, 4-tert.-Butylbenzyl, 4-(1,1,2-Trimethylpropyl)-benzyl, 2,4-Dimethylbenzyl, 4-Methoxybenzyl, 4-tert.-Butoxybenzyl oder 3,4-Dimethoxybenzyl;

$R^1$ und $R^2$ zusammen mit den Atomen, deren Substituenten sie sind, einen 5- bis 11-gliedrigen heterocyclischen Ring bedeuten, insbesondere einen Imidazolinyl-, Tetrahydro-pyrimidinyl-, Tetrahydro-1,3-diazepinyl-, Hexahydro-1,3-diazocinyl- oder Hexahydro-1,3-diazoninylgruppe, wobei diese bis zu drei der folgenden Substituenten tragen können:

$C_1$-$C_{10}$-Alkylgruppen, insbesondere die Methyl-, Ethyl-, Propyl-, Isopropyl-, Butyl-, Isobutyl-, sek.-Butyl-, tert.-Butyl-, 1,1-Dimethylpropyl- oder die 2,4,4-Trimethyl-pent-2-yl-gruppe;

$C_1$-$C_8$-Alkoxy-$C_1$-$C_8$-alkylgruppe, insbesondere die Methoxy-methyl-, Ethoxy-methyl-, Methoxy-ethyl-, tert.-Butoxy-methyl- oder die 1-Methoxy-1-methyl-ethylgruppe;

besonders bevorzugt unter den Resten $R^1$ und $R^2$ werden Imidazolinyl, Tetrahydro-pyrimidinyl, 4,4-Dimethyl-tetrahydropyrimidinyl, 5-tert-Butyl-tetrahydropyrimidinyl oder 5-Ethyl-5-propylhexahydro-1,3-diazocinyl

X $CH_2$, O, eine C-C-Einfachbindung, NH, N-($C_1$-$C_{10}$-Alkyl), insbesondere N-Methyl, N-Ethyl, N-Propyl, N-Isopropyl, N-Butyl, N-Isobutyl, N-sek.-Butyl, N-tert.-Butyl oder N-1,1-Dimethylpropyl, N-Benzyl, wobei der Phenylring noch durch $C_1$-$C_{10}$-Alkyl, insbesondere Methyl, Ethyl, Propyl, Isopropyl, Butyl, Isobutyl, sek.-Butyl, tert.-Butyl, 1,1-Dimethylethyl, 1,1-Dimethylpropyl, 2,3-Dimethylpropyl, 1,1,2-Trimethylpropyl, $C_1$-$C_{10}$-Alkoxy, insbesondere Methoxy, Ethoxy, Isopropoxy, n-Butoxy, tert.-Butoxy oder Octyloxy, $C_1$-$C_{10}$-Halogenalkyl, Trifluormethyl oder Pentafluorethyl oder Halogen, insbesondere Fluor, Chlor oder Brom, substituiert sein kann;

besonders bevorzugt unter dem Rest X werden $CH_2$, NH, N-Methyl, N-Ethyl, N-Benzyl, N-4-tert.-Butylbenzyl, N-4-(1,1,2-Trimethylpropyl)-benzyl, N-4-Methoxybenzyl, N-4-tert.-Butoxy-benzyl, N-4-Chlorbenzyl, N-4-Fluorbenzyl oder eine C-C-Einfachbindung;

n 5 bis 8, d.h. 5, 6, 7, 8.

Weiterhin bevorzugt werden Verbindungen, in denen $R^1$ und $R^2$ unabhängig voneinander Wasserstoff oder Cycloalkyl, wie oben definiert, und X NH bedeuten. Es werden weiter bevorzugt Verbindungen, in denen $R^1$ und $R^2$ zusammen Imidazolinyl oder Tetrahydropyrimidinyl und X NH oder eine Einfachbindung bedeuten.

Als Säureadditionssalze eignen sich die pflanzenverträglichen Salze von solchen Säuren, die die fungizide Wirkung von I nicht beeinträchtigen, also z. B. die Iodide, Chloride, Bromide, Sulfate, Dodecylsulfate, Nitrate, Carbonate, Phosphate, Borate, Formiate, Acetate, Propionate, Benzoate, Oxalate, Naphthalinsulfonate, Dodecylbenzolsulfonate, Lactate, Citrate und die Salze mit dem Anion des Saccharins.

Als Metallkomplexe kommen die Komplexe des Kupfers, Zinks, Zinns, Mangans, Eisens, Kobalts oder Nickels in Betracht. Vorzugsweise stellt man die Komplexe aus den freien Basen I und den mineralsauren Salzen, beispielsweise den Chloriden oder Sulfaten, der Metalle her.

Die Bis-Guanidine I sind auf verschiedenen Wegen darstellbar, und zwar bevorzugt nach den folgenden Methoden:

a) Herstellung aus Isothiuroniumsalzen und Diaminen

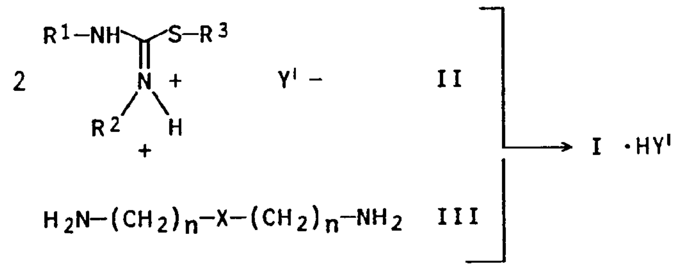

II

III

I · HY'

In diesen Formeln steht $R^3$ für eine Benzyl- oder eine $C_1$-$C_4$-Alkylgruppe, z. B. Methyl oder Ethyl, und Y' für Chlorid, Bromid, Iodid, Sulfat, Methylsulfat, Methylsulfonat oder Tosylat.

Die Ausgangsverbindungen II und III sind bekannt oder auf bekannte Weise erhältlich (bezüglich der Isothiuroniumsalze s. Houben-Weyl, Methoden der Organischen Chemie, 4. Auflage, Bd. IX, S. 900ff).

Die im Formelschema aufgezeigte an sich bekannte Umsetzung von Isothiuroniumsalzen mit Diaminen (z. B. A. V. Bogatskii et al., Khim.-Farm. Zh. **17** (1983) 308; CA **98** 215574z) zu Bis-Guanidin-Verbindungen I·HY' erfolgt bevorzugt in polaren Lösungsmitteln wie Alkoholen, Ketonen, Ethern, Nitrilen, Dimethylsulfoxid, N-Methylpyrrolidon, Dimethylformamid oder Dimethylacetamid.

Bei der Durchführung der Reaktion setzt man im allgemeinen pro Mol an Diaminoverbindung III 2,0 bis 4,0 Mol, besonders bevorzugt 2,0 Mol an Isothiuroniumsalz II ein. Zusätzlich kann noch ein tertiäres Amin wie Triethylamin als Hilfsbase zugegeben werden. Bevorzugt setzt man in diesem Fall äquimolare Mengen der Hilfsbase, bezogen auf das Isothiuroniumsalz, ein.

Die Reaktionstemperaturen können zwischen 20°C und der Siedetemperatur des Lösungsmittels liegen, vorzugsweise arbeitet man zwischen 60 und 130°C.

Die Umsetzung erfolgt bevorzugt bei Normaldruck.

Durch Anionenaustausch lassen sich Salze mit anderen Anionen - oder - bei Ersatz durch Hydroxyl-Ionen - die freien Basen I erhalten.

b) Herstellung aus Aminoiminomethansulfonsäuren und Diaminen

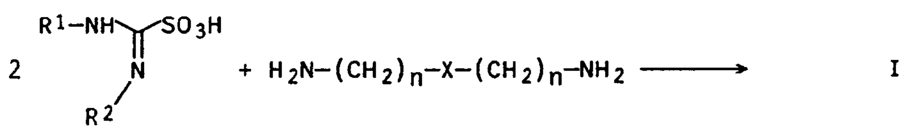

IV III

I

Die Ausgangverbindungen IV sind bekannt bzw. lassen sich auf bekannte Weise aus Thioharnstoffderivaten darstellen (z. B.C.A. Maryanoff et al., J. Org. Chem., **51** (1986) 1882).

Für die Umsetzung empfehlen sich Temperaturen zwischen 0°C und der Siedetemperatur des Lösungsmittels.

Bezüglich der Mengenverhältnisse und des Druckes gelten die Angaben für Methode a).

Bezüglich der Lösungsmittel gelten die Angaben für Methode a), ganz besonders bevorzugt ist Acetonitril.

c) Herstellung aus Carbodiimiden und Diaminen

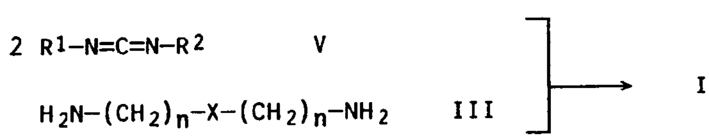

V

III

I

Die Ausgangsverbindungen V sind bekannt oder lassen sich in Analogie zu bekannten Verfahren herstellen (vgl. z. B.M. Mikolaiczyk, Tetrahedron **37** (1981) 233).

Für die Umsetzung empfehlen sich pro Mol an Diaminoverbindung III 2 Mol an Carbodiimidverbindung V einzusetzen, bevorzugt arbeitet man über das Verhältnis 1 : 2 hinaus mit einem geringen Überschuß der Amin- oder Carbodiimidkomponente, bis etwa 10 %.

Als Lösungsmitttel für die Umsetzungen kommen Kohlenwasserstoffe wie Hexan oder Toluol, kurzkettige Alkohole wie Isopropanol oder tert.-Butanol, Amide wie Dimethylformamid oder Nitrile wie Acetonitril in Frage.

Bezüglich der Temperatur und des Druckes gelten die Angaben für Methode a).

d) Herstellung aus Imidocarbonaten und Aminen

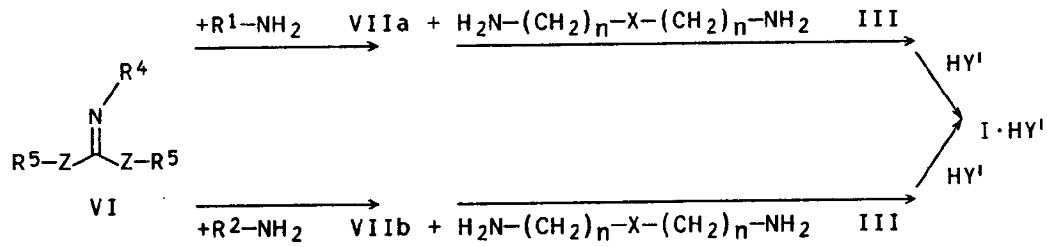


In dieser Formel steht im Falle von Z = O $R^4$ für eine Cyan-, Benzoyl- oder Methansulfonsäuregruppe und $R^5$ für eine Phenylgruppe oder im Falle von Z = S $R^4$ für eine Cyan- und $R^5$ für eine Methylgruppe.

Die an sich bekannte Umsetzung von Imidocarbonaten mit zwei Aminen (vgl. z.B. A. Buschauer, Arzneim.-Forsch./Drug. Res., **37** (1987) 1003, 1008; Arch. Pharm., **321** (1988) 281) erfolgt in zwei getrennten Stufen. Die Reaktion des Imidocarbonats mit dem ersten Amin wird bevorzugt in einem chlorierten Kohlenwasserstoff wie Dichlormethan, einem Ether wie Diethylether oder Tetrahydrofuran, einem kurzkettigen Alkohol wie Methanol oder Isopropanol, einer Wasser/Alkohol-Mischung wie Wasser/Methanol oder einem Nitril wie Acetonitril durchgeführt. Die weitere Umsetzung des Produktes mit dem zweiten Amin erfolgt dann in einem polaren Lösungsmittel wie Methanol, Ethanol, Isopropanol, tert.-Butanol, Pyridin oder Acetonitril.

Für die Umsetzungen empfehlen sich pro Mol an Imidocarbonatverbindung VI 1,0 Mol an Aminverbindung VIIa bzw. VIIb und 0,5 Mol an Diaminverbindung III einzusetzen, bevorzugt arbeitet man mit einem geringen Überschuß der Aminkomponente, bis etwa 10 %.

Bezüglich der Temperatur und des Druckes gelten die Angaben für Methode a).

Die Hydrolyse der erhaltenen Verbindungen erfolgt auf bekannte Weise, vorteilhaft in einer Mineralsäure oder aber auch einer organischen Carbonsäure bei z. B. 70 bis 120°C. Bevorzugt verwendet man 2 bis 10 M Salzsäure oder 50 bis 80%ige Essigsäure.

Man erhält die Chlorid- oder Acetatsalze der Verbindungen I mit $R^1$ oder $R^2$ gleich Wasserstoff.

Bezüglich der Darstellung der freien Basen I gelten die Angaben für Methode a).

e) Herstellung aus Bromcyan und Aminen

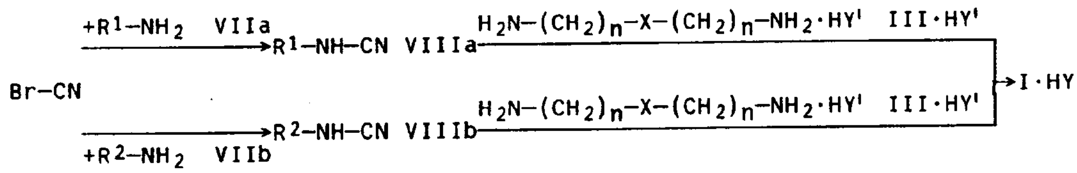

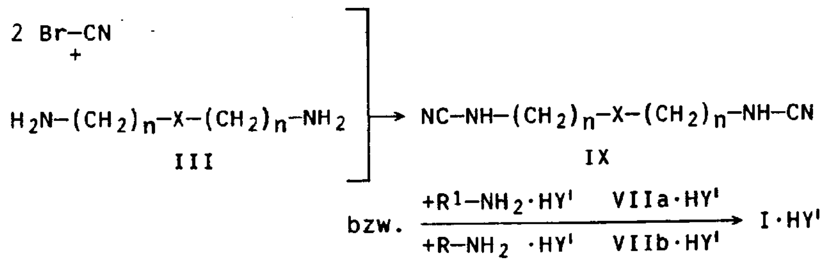


In diesen Formeln bedeutet Y' bevorzugt Chlorid.

Die aus Houben-Weyl, Methoden der Org. Chemie, 4. Auflage, Bd. E4, S. 981, an sich bekannte Umsetzung von Bromcyan mit Aminen zu N-substituierten Cyanamiden erfolgt bevorzugt im Zweiphasensystem, z.B. Wasser/Dichlormethan, oder aber wasserfrei in inerten Lösungsmitteln wie Diethylether, Tetrahydrofuran, Dioxan, Dichlormethan oder Toluol.

Für die Umsetzungen empfehlen sich Temperaturen von -20 bis +10°C.

Bezüglich der Mengenverhältnisse und des Druckes gelten die Angaben für Methode d).

Die aus Houben-Weyl, Methoden der Org. Chemie, 4. Auflage, Bd. E4, S. 609, an sich bekannte Umsetzung der N-substituierten Cyanamide VIIIa, VIIIb und IX mit den Hydrochloriden der Amine III, VIIa und VIIb erfolgt bevorzugt lösungsmittelfrei bei Temperaturen von beispielsweise 130 bis 250°C.

Bezüglich der eingesetzten Mengen, des Druckes oder der Darstellung der freien Basen I gelten die Angaben für Methode d).

Man erhält die Verbindungen I mit $R^1$ oder $R^2$ gleich Wasserstoff.

Die Verbindungen der Formel I und ihre Salze und Metallkomplexe eignen sich als Fungizide bei guter Pflanzenverträglichkeit.

Herstellungsbeispiele

Beispiel 1, nach Methode a), (Verbindung 11a)

1,13-Bis-(3,3-dimethyl-cyclohexyl)-guanidino-7-aza-tridecan-di-hydroiodid

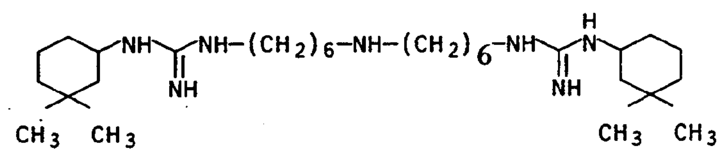 · 2 HJ

Eine Mischung aus 16,4 g (0,050 mol) 3,3-Dimethyl-cyclohexyl-S-methyl-isothiuroniumiodid, 5,3 g (0,025 mol) 1,13-Diamino-7-aza-tridecan, 5 g (0,050 mol) Triethylamin und 15 g Molekularsieb (4Å) in 200 ml wasserfreiem Acetonitril wurde unter einer Stickstoffatmosphäre 48 Stunden lang unter Abspaltung von Methanthiol auf Rückflußtemperatur erhitzt. Nach heißer Filtration wurde die Verbindung von der Lösung abgetrennt.

Ausbeute: 93 % d. Theorie; Fp.: 120°C.

Vorstufe A1

3,3-Dimethyl-cyclohexyl-S-methyl-isothiuroniumiodid

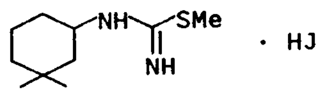

Eine Lösung von 63 g (0,339 mol) 3,3-Dimethyl-cyclohexylthioharnstoff und 48,1 g (0,339 mol) Iodmethan in 100 ml Methanol wurde 1 Stunde auf Rückflußtemperatur erhitzt. Nach dem Abdestillieren des Lösungsmittels im Vakuum blieb ein gelber harzartiger Festkörper zurück.

Ausbeute: 58 % d. Theorie; Fp.: 80°C.

Vorstufe A2

3,3-Dimethyl-cyclohexyl-thioharnstoff

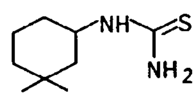

Zu einer Lösung von 33 g (0,44 mol) Ammoniumrhodanid in 100 ml absolutem Aceton wurde innerhalb von 10 Minuten 59 g (0,42 mol) Benzoylchlorid getropft. Nach 10minütigem Rühren bei Rückflußtemperatur tropfte man 50,8 g (0,40 mol) 3,3-Dimethylcyclohexylamin zu, erhitzte weitere 20 Minuten zum Rückfluß und rührte das Reaktionsgemisch in 500 ml Eiswasser. Der ausgefallene Feststoff wurde abgesaugt, mit Wasser gewaschen, in einem heißen Gemisch aus 500 ml 10%iger Natronlauge und 125 ml Ethanol gelöst und 30 Minuten zum Rückfluß erhitzt. Anschließend wurde die Reaktionsmischung mit Eiswasser verdünnt, mit konzentrierter Salzsäure zunächst auf pH = 1 und schließlich mit festem Natriumhydrogencarbonat auf pH = 9 eingestellt. Der resultierende Niederschlag wurde abgesaugt, mit Wasser gewaschen und bei 80°C im Vakuum getrocknet.

Ausbeute: 85 % d. Th.; Fp.: 153°C.

Beispiel 2 (Verbindung 18a)

1,17-Bis-cyclohexylguanidino-9-aza-heptadecan-tri-acetat

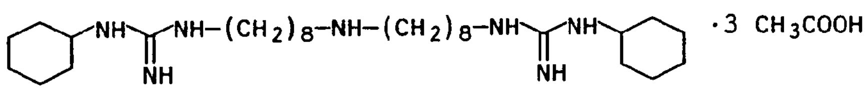

10,2 g (0,011 mol) 1,17-Bis-cyclohexylguanidino-9-aza-heptadecan-dihydroiodid in 150 ml Methanol/Wasser (1 : 1) wurden über eine Säule mit 250 g Ionenaustauscher ($OH^-$-Form) filtriert. Nach Aufarbeitung wurde die erhaltene freie Guanidin-Base in Methanol gelöst und mit einem Überschuß Eisessig in das Tri-acetat übergeführt.

Beispiel 3, nach Methode a) (Verbindung 16a)

1,17-Bis-cyclohexylguanidino-9-aza-heptadecan-di-hydroiodid

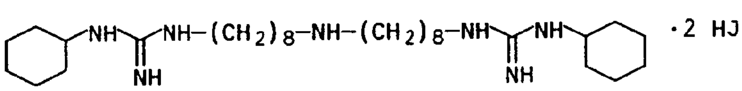

Eine Mischung aus 30,0 g (0,10 mol) Cyclohexyl-S-methylisothiuroniumiodid, 13,6 g (0,05 mol) 1,17-Diamino-9-aza-heptadecan, 10,0 g (0,10 mol) Triethylamin und 20 g Molekularsieb (4 Å) in 300 ml wasserfreiem Acetonitril wurde unter einer Stickstoffatmosphäre 5 Tage lang unter Abspaltung von Methanthiol auf Rückflußtemperatur erhitzt. Nach heißer Filtration wurde wie üblich aufgearbeitet.

Ausbeute: 43 % d. Theorie; Fp.: 170°C.

Vorstufe B1

Cyclohexyl-S-methyl-isothiuroniumiodid

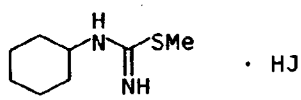

Eine Lösung von 62,0 g (0,392 mol) Cyclohexyl-thioharnstoff und 55,7 g (0,392 mol) Iodmethan in 300 ml Methanol wurde 1 Stunde auf Rückflußtemperatur erhitzt. Nach dem Abkühlen auf Raumtemperatur wurde das Produkt durch Zugabe von Methyl-tert.-butyl-ether ausgefällt, abgesaugt, mit Methyl-tert.-butyl-ether und Hexan gewaschen und schließlich bei 50°C im Vakuum getrocknet.

Ausbeute: 68 % d. Theorie; Fp.: 115 - 120°C.

Vorstufe B2

Cyclohexyl-thioharnstoff

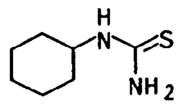

Zu einer Lösung von 165,0 g (2,2 mol) Ammoniumrhodanid in 500 ml absolutem Aceton wurde innerhalb von 10 Minuten 295,5 g (2,1 mol) Benzoylchlorid getropft. Nach 10minütigem Rühren bei Rückflußtemperatur tropfte man 198,9 g (2,0 mol) Cyclohexylamin zu und erhitzte weitere 20 Minuten zum Rückfluß. Danach rührte man das Reaktionsgemisch in 4 l Eiswasser und dekantierte das Wasser vom ölig anfallenden Produkt ab. Der Rückstand wurde in einem heißen Gemisch aus 2 l 10%iger Natronlauge und 500 ml Ethanol gelöst und 30 Minuten zum Rückfluß erhitzt. Anschließend wurde die Reaktionsmischung mit Eiswasser verdünnt, mit konzentrierter Salzsäure zunächst auf pH = 1 und schließlich mit festem Natriumhydrogencarbonat auf pH = 9 eingestellt. Der resultierende Niederschlag wurde abgesaugt, mit Wasser gewaschen und bei 80°C im Vakuum getrocknet.

Ausbeute: 78 % d. Th.; Fp.: 135°C.

Beispiel 4, nach Methode c) (Verbindung 14a)

1,13-Bis-(N,N'-dicyclohexyl)guanidino-7-aza-tridecan-tri-hydrochlorid

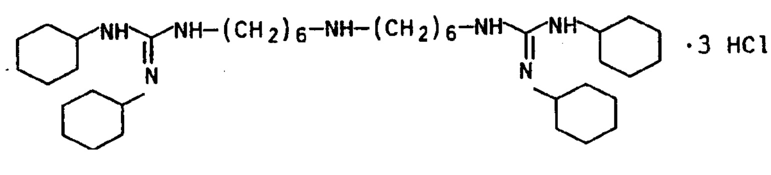

Eine Mischung aus 49,4 g (0,24 mol) Dicyclohexylcarbodiimid, 21,5 g (0,10 mol) 1,13-Diamino-7-aza-tridecan und 400 ml wasserfreiem tert.-Butanol wurde 12 Stunden lang auf Rückflußtemperatur erhitzt. Nach Isolierung versetzte man das ölige Produkt mit methanolischer Salzsäure und destillierte das Lösungsmittel im Vakuum ab, woraufhin ein farbloser Festkörper zurückblieb.

Ausbeute: 26 % d. Theorie; Fp.: 140°C

Beispiel 5 (Verbindung 35b)

1,12-Bis-(2-amino-3,4,5,6-tetrahydro-pyrimidinyl)-dodecan-di-acetat

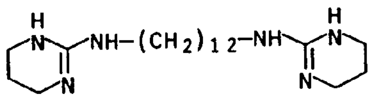

· 2 $CH_3COOH$

30,0 g (0,048 mol) 1,12-Bis-(2-amino-3,4,5,6-tetrahydropyrimidinyl)-dodecan-di-hydroiodid in 300 ml Methanol/Wasser (1 : 1) wurden über eine Säule mit 250 g Ionenaustauscher ($OH^-$-Form) filtriert. Nach Aufarbeitung wurde die erhaltene freie Guanidin-Base in Methanol gelöst und mit einem Überschuß Eisessig in das Di-acetat übergeführt.

Beispiel 6, nach Methode a) (Verbindung 34b)

1,12-Bis-(2-amino-3,4,5,6-tetrahydro-pyrimidinyl)-dodecan-di-hydroiodid

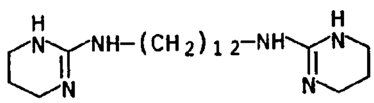

·2 HJ

Eine Mischung aus 103,3 g (0,4 mol) 2-S-Methyl-3,4,5,6-tetrahydro-pyrimidinium-iodid, 40,0 g (0,2 mol) 1,12-Diamino-dodecan, 20,2 g (0,2 mol) Triethylamin und 60 g Molekularsieb (4 Å) in 1 l wasserfreiem Acetonitril wurde unter einer Stickstoffatmosphäre 16 Stunden lang unter Abspaltung von Methanthiol auf Rückflußtemperatur erhitzt. Nach heißer Filtration wurde wie üblich aufgearbeitet.

Ausbeute: 81 % d. Theorie eines braunen Öls

IR (Film): 3217, 2925, 2853, 1642, 1315 $cm^{-1}$

Vorstufe D1

2-S-Methyl-3,4,5,6-tetrahydro-pyrimidinyl-isothiuronium-iodid

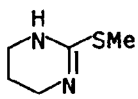

· HJ

Eine Lösung von 50,0 g (0,431 mol) Perhydro-pyrimidin-thion und 61,2 g (0,431 mol) Iodmethan in 200 ml Methanol wurde 1 Stunde auf Rückflußtemperatur erhitzt. Nach dem Abkühlen auf Raumtemperatur wurde das Produkt durch Zugabe von Methyl-tert.-butyl-ether augefällt, abgesaugt, mit Methyl-tert.-butyl-ether gewaschen und getrocknet.

Ausbeute: 94 % d. Theorie; Fp.: 135°C

Tabelle 1

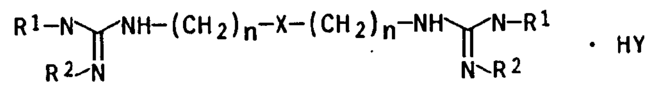

| Verb.-Nr. | $R^1$ | $R^2$ | X | n | HY | Fp./IR(Film) [$cm^{-1}$] |
|---|---|---|---|---|---|---|
| 1a | Cyclohexyl | H | NH | 6 | HCl | 125°C |
| 2a | Cyclohexyl | H | NH | 6 | $CH_3COOH$ | 100°C |
| 3a | Cyclohexyl | H | NH | 6 | $C_2H_2O_4$ | 140°C |
| 4a | Cyclohexyl | H | NH | 6 | - | |
| 5a | Cyclopentyl | H | NH | 6 | - | |
| 6a | Cyclooctyl | H | NH | 6 | - | |
| 7a | Cyclododecyl | H | NH | 6 | - | |
| 8a | 4-tert.-Butyl-cyclohexyl | H | NH | 6 | - | |
| 9a | 4-Isopropyl-cyclohexyl | H | NH | 6 | - | |
| 10a | 3-Trifluormethyl-cyclohexyl | H | NH | 6 | - | |
| 11a | 3,3-Dimethyl-cyclohexyl | H | NH | 6 | HJ | 120°C |
| 12a | Cyclohexyl | Cyclohexyl | NH | 6 | - | 2926, 2851, 1639, 1448, |
| 13a | Cyclohexyl | Cyclohexyl | NH | 6 | $CH_3COOH$ | 3205, 3085, 2930, 2855, 1612, 1573, 1450, 1400, 1366, 1246 |

Tabelle 1 - Fortsetzung -

| Verb.-Nr. | $R^1$ | $R^2$ | X | n | HY | Fp./IR(Film) [$cm^{-1}$] |
|---|---|---|---|---|---|---|
| 14a | Cyclohexyl | Cyclohexyl | NH | 6 | HCl | 140°C |
| 15a | Cyclohexyl | H | NH | 8 | - | 3279, 3185, 2929, 2854, 1652 |
| 16a | Cyclohexyl | H | NH | 8 | HJ | 170 - 172°C |
| 17a | Cyclohexyl | H | NH | 8 | HCl | 110°C |
| 18a | Cyclohexyl | H | NH | 8 | $CH_3COOH$ | 98 - 100°C |
| 19a | Cyclohexyl | H | NH | 8 | $C_2H_2O_4$ | 165 - 167°C |
| 20a | Cyclopentyl | H | NH | 8 | - | |
| 21a | Cyclooctyl | H | NH | 8 | - | |
| 22a | Cyclododecyl | H | NH | 8 | - | |
| 23a | 4-tert.-Butyl-cyclohexyl | H | NH | 8 | - | |
| 24a | 4-Isopropyl-cyclohexyl | H | NH | 8 | - | |
| 25a | 3-Trifluormethyl-cyclohexyl | H | NH | 8 | - | |
| 26a | 3,3-Dimethyl-cyclohexyl | H | NH | 8 | - | |
| 27a | Cyclohexyl | Cyclohexyl | NH | 8 | - | |
| 28a | Cyclohexyl | H | Einfachbdg. | 5 | | |
| 29a | Cyclopentyl | H | Einfachbdg. | 5 | | |
| 30a | Cyclooctyl | H | Einfachbdg. | 5 | | |
| 31a | Cyclododecyl | H | Einfachbdg. | 5 | - | |
| 32a | 4-tert.-Butyl-cyclohexyl | H | Einfachbdg. | 5 | - | |
| 33a | 4-Isopropyl-cyclohexyl | H | Einfachbdg. | 5 | - | |
| 34a | 3-Trifluormethyl-cyclohexyl | H | Einfachbdg. | 5 | - | |
| 35a | 3,3-Dimethyl-cyclohexyl | H | Einfachbdg. | 5 | - | |
| 36a | Cyclohexyl | Cyclohexyl | Einfachbdg. | 5 | - | |

Tabelle 1 - Fortsetzung -

| Verb.-Nr. | $R^1$ | $R^2$ | X | n | HY | Fp./IR(Film) [$cm^{-1}$] |
|---|---|---|---|---|---|---|
| 37a | Cyclohexyl | H | $CH_2$ | 5 | - | |
| 38a | Cyclopentyl | H | $CH_2$ | 5 | - | |
| 39a | Cyclooctyl | H | $CH_2$ | 5 | - | |
| 40a | Cyclododecyl | H | $CH_2$ | 5 | - | |
| 41a | 4-tert.-Butyl-cyclohexyl | H | $CH_2$ | 5 | - | |
| 42a | 4-Isopropyl-cyclohexyl | H | $CH_2$ | 5 | - | |
| 43a | 3-Trifluormethyl-cyclohexyl | H | $CH_2$ | 5 | - | |
| 44a | 3,3-Dimethyl-cyclohexyl | H | $CH_2$ | 5 | - | |
| 45a | Cyclohexyl | Cyclohexyl | $CH_2$ | 5 | - | |
| 46a | Cyclohexyl | H | Einfachbdg. | 6 | - | |
| 47a | Cyclopentyl | H | Einfachbdg. | 6 | - | |
| 48a | Cyclooctyl | H | Einfachbdg. | 6 | - | |
| 49a | Cyclododecyl | H | Einfachbdg. | 6 | - | |
| 50a | 4-tert.-Butyl-cyclohexyl | H | Einfachbdg. | 6 | - | |
| 51a | 4-Isopropyl-cyclohexyl | H | Einfachbdg. | 6 | - | |
| 52a | 3-Trifluormethyl-cyclohexyl | H | Einfachbdg. | 6 | - | |
| 53a | 3,3-Dimethyl-cyclohexyl | H | Einfachbdg. | 6 | - | |
| 54a | Cyclohexyl | Cyclohexyl | Einfachbdg. | 6 | - | |
| 55a | p-tert.-Butylbenzyl | H | NH | 6 | - | |
| 57a | p-tert.-Butylbenzyl | Cyclohexyl | NH | 6 | - | |
| 58a | p-(1,1,2-Trimethylpropyl)benzyl | H | NH | 6 | - | |
| 59a | p-(1,1,2-Trimethylpropyl)benzyl | Cyclooctyl | NH | 6 | - | |

Tabelle 1 - Fortsetzung -

| Verb.-Nr. | $R^1$ | $R^2$ | X | n | HY | Fp./IR(Film) [$cm^{-1}$] |
|---|---|---|---|---|---|---|
| 60a | p-tert.-Butoxybenzyl | H | NH | 6 | - | |
| 61a | p-tert.-Butoxybenzyl | Cyclohexyl | NH | 6 | - | |
| 62a | p-tert.-Butylbenzyl | H | NH | 8 | - | |
| 63a | p-tert.-Butylbenzyl | Cyclohexyl | NH | 8 | - | |
| 64a | p-tert.-Butylbenzyl | Cyclooctyl | NH | 8 | - | |
| 65a | p-Isopropylbenzyl | H | NH | 8 | - | |
| 67a | p-Methoxybenzyl | H | NH | 8 | - | |
| 69a | p-tert.-Butylbenzyl | H | Einfachbdg. | 5 | - | |
| 70a | p-tert.-Butylbenzyl | Cyclohexyl | Einfachbdg. | 5 | - | |
| 71a | p-Ethoxybenzyl | H | Einfachbdg. | 5 | - | |
| 73a | p-tert.-Butylbenzyl | H | $CH_2$ | 5 | - | |
| 74a | p-tert.-Butylbenzyl | Cyclohexyl | $CH_2$ | 5 | - | |
| 75a | p-tert.-Butylbenzyl | Cyclooctyl | $CH_2$ | 5 | - | |
| 76a | p-(1,1,2-Trimethylpropyl)benzyl | H | $CH_2$ | 5 | - | |
| 77a | p-(1,1,2-Trimethylpropyl)benzyl | Cyclooctyl | $CH_2$ | 5 | - | |
| 78a | p-tert.-Butoxybenzyl | H | $CH_2$ | 5 | - | |
| 80a | p-tert.-Butylbenzyl | H | Einfachbdg. | 6 | - | |

Tabelle 1 - Fortsetzung -

| Verb.-Nr. | $R^1$ | $R^2$ | X | n | HY | Fp./IR(Film) [$cm^{-1}$] |
|---|---|---|---|---|---|---|
| 82a | p-tert.-Butylbenzyl | Cyclohexyl | Einfachbdg. | 6 | - | |
| 83a | p-tert.-Butoxybenzyl | H | Einfachbdg. | 6 | - | |
| 84a | p-tert.-Butoxybenzyl | Cyclohexyl | Einfachbdg. | 6 | - | |
| 85a | Cyclohexyl | H | NMe | 6 | - | |
| 86a | Cyclohexyl | H | $N-CH_2Ph$ | 8 | - | |
| 87a | p-tert.-Butylbenzyl | H | N-Me | 6 | - | |
| 88a | p-tert.-Butylbenzyl | H | $N-CH_2$ 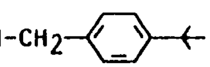 | 8 | - | |

Tabelle 2

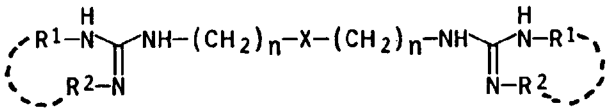 · HY

| Verb.-Nr. | 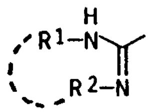 | X | n | HY | Fp./IR(Film) [$cm^{-1}$] |
|---|---|---|---|---|---|
| 1b | 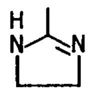 | NH | 6 | HCl | 195°C |
| 2b | 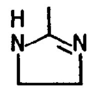 | NH | 6 | $CH_3COOH$ | 3165, 3051, 2933, 2858, 1679, 1560, 1471, 1402, 1286 |
| 3b | 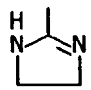 | NH | 6 | $C_2H_2O$ | 100°C |
| 4b | 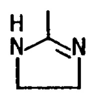 | NH | 6 | - | |
| 5b | 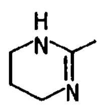 | NH | 6 | HJ | 3225, 2930, 1643, 1316 |
| 6b | 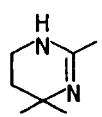 | NH | 6 | - | |

Tabelle 2 - Fortsetzung -

| Verb.-Nr. | 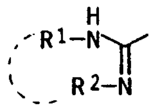 | X | n | HY | Fp./IR(Film) [$cm^{-1}$] |
|---|---|---|---|---|---|
| 7b | 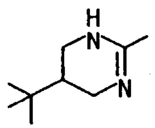 | NH | 6 | - | |
| 8b | 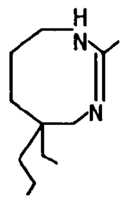 | NH | 6 | - | |
| 9b | 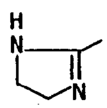 | NH | 8 | - | |
| 10b | 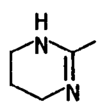 | NH | 8 | HJ | 3218, 2926, 2854, 1643, 1316 |
| 11b | 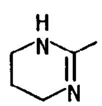 | NH | 8 | HCl | 3308, 3236, 3160, 3057, 2929, 2853, 2812, 1654, 1634, 1314 |

Tabelle 2 - Fortsetzung -

| Verb.-Nr. | 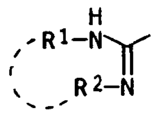 | X | n | HY | Fp./IR(Film) [$cm^{-1}$] |
|---|---|---|---|---|---|
| 12b | 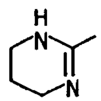 | NH | 8 | $CH_3COOH$ | 3227, 3160, 2927, 2854, 1667, 1567, 1441, 1398, 1318 |
| 13b | 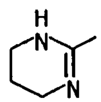 | NH | 8 | $C_2H_2O_4$ | 140°C |
| 14b | 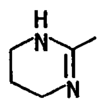 | NH | 8 | - | |
| 15b | 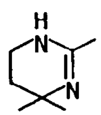 | NH | 8 | - | |
| 16b | 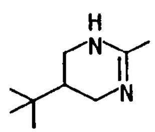 | NH | 8 | - | |

Tabelle 2 - Fortsetzung -

| Verb.-Nr. | 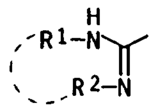 | X | n | HY | Fp./IR(Film) [$cm^{-1}$] |
|---|---|---|---|---|---|
| 17b | 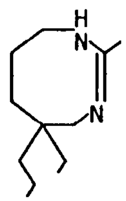 | NH | 8 | - | |
| 18b | 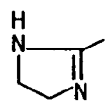 | Einfachbdg. | 5 | - | |
| 19b | 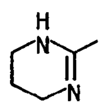 | Einfachbdg. | 5 | - | |
| 20b | 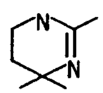 | Einfachbdg. | 5 | - | |
| 21b | 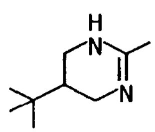 | Einfachbdg. | 5 | - | |

Tabelle 2 - Fortsetzung -

| Verb.-Nr. | 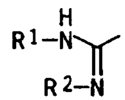 | X | n | HY | Fp./IR(Film) [$cm^{-1}$] |
|---|---|---|---|---|---|
| 22b | 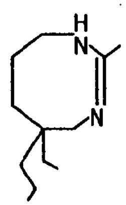 | Einfachbdg. | 5 | - | |
| 23b | 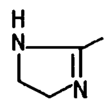 | $CH_2$ | 5 | - | |
| 24b | 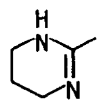 | $CH_2$ | 5 | - | |
| 25b | 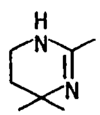 | $CH_2$ | 5 | - | |
| 26b | 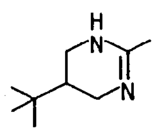 | $CH_2$ | 5 | - | |

Tabelle 2 - Fortsetzung -

| Verb.-Nr. | 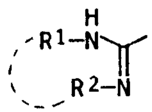 | X | n | HY | Fp./IR(Film) [$cm^{-1}$] |
|---|---|---|---|---|---|
| 27b | 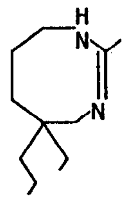 | $CH_2$ | 5 | - | |
| 28b | 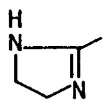 | Einfachbdg. | 6 | - | 3284, 2916, 2849, 1637, 1612, 1556, 1470, 1262 |
| 29b | 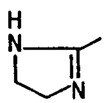 | Einfachbdg. | 6 | HJ | 197°C |
| 30b | 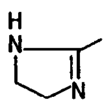 | Einfachbdg. | 6 | HCl | 3290, 3142, 3063, 3001, 2984, 2926, 2862, 2853, 1674, 1590 |
| 31b | 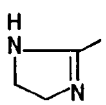 | Einfachbdg. | 6 | $CH_3COOH$ | 2926, 2854, 1677, 1560, 1396, 1285 |

Tabelle 2 - Fortsetzung -

| Verb.-Nr. | 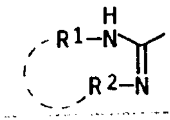 | X | n | HY | Fp./IR(Film) [$cm^{-1}$] |
|---|---|---|---|---|---|
| 32b | 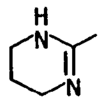 | Einfachbdg. | 6 | - | 156 - 158°C |
| 33b | 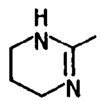 | Einfachbdg. | 6 | HCl | 3214, 3067, 2926, 2854, 1646, 1317 |
| 34b | 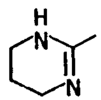 | Einfachbdg. | 6 | HJ | 3217, 2925, 2853, 1642, 1315 |
| 35b | 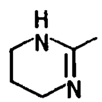 | Einfachbdg. | 6 | $CH_3COOH$ | 3231, 2928, 2855, 1660, 1558, 1275 |
| 36b | 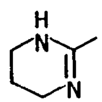 | Einfachbdg. | 6 | $C_2H_2O_4$ | 140 - 142°C |
| 37b | 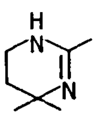 | Einfachbdg. | 6 | - | |

Tabelle 2 - Fortsetzung -

| Verb.-Nr. | 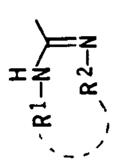 | X | n | HY | Fp./IR(Film) [$cm^{-1}$] |
|---|---|---|---|---|---|
| 38b | 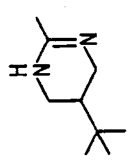 | Einfachbdg. | 6 | - | |
| 39b | 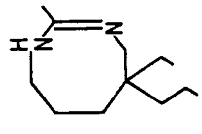 | Einfachbdg. | 6 | - | |
| 40b | 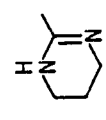 | N-Me | 8 | - | |
| 41b | 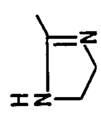 | N-CH2- 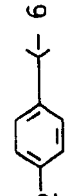 | 6 | - | |

Die Verbindungen eignen sich als Fungizide.

Die fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Nr. 12a und 10 Gew.-Teilen N-Methyl-$\alpha$-pyrrolidon, die zur Anwendung in Form kleinster Tropfen geeignet ist;

II. eine Mischung aus 20 Gew.-Teilen der Verbindung Nr. 14a, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 30b, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 31b, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 35b, 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 31b und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 12a, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 14a, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 14a, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

## Anwendungsbeispiele

Als Vergleichswirkstoff wurde Guazatine-Triacetat (A) - bekannt aus GB 1 114 155 - benutzt.

### Anwendungsbeispiel 1

Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs

abermals für 16 Stunden in der feuchten Kammer aufgestellt. Dann erfolgte die Beurteilung des Ausmaßes des Pilzausbruchs auf den Blattunterseiten.

Das Ergebnis zeigt, daß die Wirkstoffe 12a, 14a, 30b und 31b bei der Anwendung als 0,025%ige (Gew.-%) Spritzbrühe eine bessere fungizide Wirkung zeigen (95 %) als der bekannte Vergleichswirkstoff A (65 %).

## Patentansprüche

**1.** Substituierte Bis-Guanidine der allgemeinen Formel I

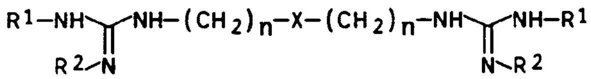

I

in der

R¹ und R² unabhängig voneinander Wasserstoff, $C_3$-$C_{10}$-Alkenyl, $C_3$-$C_{10}$-Alkinyl, $C_1$-$C_8$-Alkoxy-$C_2$-$C_8$-alkyl, $C_3$-$C_{10}$-Halogenalkenyl; oder eine Cycloalkylgruppe mit 5 bis 12 Kohlenstoffatomen im Ring, die bis zu drei der folgenden Substituenten
Hydroxyl, $C_1$-$C_{10}$-Alkyl, $C_1$-$C_{10}$-Alkoxy, $C_1$-$C_{10}$-Halogenalkyl und $C_1$-$C_8$-Alkoxy-$C_1$-$C_8$-alkyl tragen kann;
oder eine Benzylgruppe, die durch bis zu drei $C_1$-$C1_0$-Alkyl- oder $C_1$-$C_{10}$-Alkoxy-Gruppen substituiert sein kann, wobei der Substituent noch eine Hydroxyl- oder $C_1$-$C_6$-Alkoxygruppe tragen kann; oder die Benzylgruppe durch eine $C_1$-$C_{10}$-Halo-genalkyl-Gruppe substituiert ist,
mit der Maßgabe, daß nur einer der beiden Substituenten $R^1$ oder $R^2$ Wasserstoff sein kann; oder

R¹ und R² zusammen mit den Atomen, deren Substituenten sie sind, einen 5- bis 11-gliedrigen heterocyclischen Ring bedeuten, der durch $C_1$-$C_{10}$-Alkyl oder $C_1$-$C_8$-Alkoxy-$C_1$-$C_8$-alkyl ein- bis dreifach substituiert sein kann;

X $CH_2$, O, eine C-C-Einfachbindung, NH, N-($C_1$-$C_{10}$-Alkyl) oder N-Benzyl, wobei der Phenylring des Benzylrestes einfach bis dreifach durch $C_1$-$C_{10}$-Alkyl, $C_1$-$C_{10}$-Alkoxy, $C_1$-$C_{10}$-Halogen-alkyl oder Halogen substituiert sein kann, und

n 5 bis 8 bedeuten,

sowie ihre pflanzenverträglichen Säureadditionssalze und ihre Metallkomplexe.

**2.** Verfahren zur Herstellung der Bis-Guanidine der Formel I

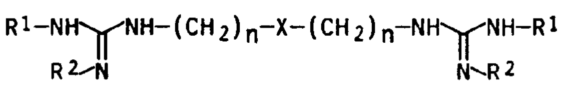

I

in der

R¹ und R² unabhängig voneinander Wasserstoff, $C_3$-$C_{10}$-Alkenyl, $C_3$-$C_{10}$-Alkinyl, $C_1$-$C_8$-Alkoxy-$C_2$-$C_8$-alkyl, $C_3$-$C_{10}$-Halogenalkenyl; oder eine Cycloalkylgruppe mit 5 bis 12 Kohlenstoffatomen im Ring, die bis zu drei der folgenden Substituenten
Hydroxyl, $C_1$-$C_{10}$-Alkyl, $C_1$-$C_{10}$-Alkoxy, $C_1$-$C_{10}$-Halogenalkyl und $C_1$-$C_8$-Alkoxy-$C_1$-$C_8$-alkyl tragen kann;
oder eine Benzylgruppe, die durch bis zu drei $C_1$-$C_{10}$-Alkyl- oder $C_1$-$C_{10}$-Alkoxy-Gruppen substituiert sein kann, wobei der Substituent noch eine Hydroxyl- oder $C_1$-$C_6$-Alkoxygruppe tragen kann; oder die Benzylgruppe durch eine $C_1$-$C_{10}$-Halogenalkyl-Gruppe substituiert ist,
mit der Maßgabe, daß nur einer der beiden Substituenten $R^1$ oder $R^2$ Wasserstoff sein kann; oder

R¹ und R² zusammen mit den Atomen, deren Substituenten sie sind, einen 5- bis 11-gliedrigen heterocyclischen Ring bedeuten, der durch $C_1$-$C_{10}$-Alkyl oder $C_1$-$C_8$-Alkoxy-$C_1$-$C_8$-alkyl ein- bis dreifach substituiert sein kann;

X CH$_2$, O, eine C-C-Einfachbindung, NH, N-($C_1$-$C_{10}$-Alkyl) oder N-Benzyl, wobei der Phenylring des Benzylrestes einfach bis dreifach durch $C_1$-$C_{10}$-Alkyl, $C_1$-$C_{10}$-Alkoxy, $C_1$-$C_{10}$-Halogen-alkyl oder Halogen substituiert sein kann,

n 5 bis 8 bedeuten,

dadurch gekennzeichnet, daß man

a) ein Isothiuronium-Salz II

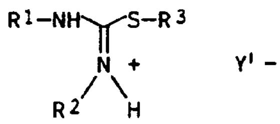

II

mit einem Diamin III

$$H_2N\text{-}(CH_2)_n\text{-}X\text{-}(CH_2)_n\text{-}NH_2 \qquad III$$

umsetzt, wobei $R^3$ eine Benzyl- oder $C_1$-$C_4$-Alkylgruppe bedeutet und Y' ein Anion, z. B. Halogenid, Sulfat, Methylsulfat, $C_1$-$C_4$-Alkylsulfonat oder p-Tolylsulfonat bedeutet,

oder

b) eine Aminoiminomethansulfonsäure IV

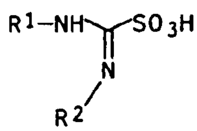

IV

mit dem Diamin III umsetzt

oder

c) ein Carbodiimid V

$$R^1\text{-}N=C=N\text{-}R^2 \qquad IV$$

mit dem Diamin III umsetzt

oder,

falls einer der Substituenten $R^1$ oder $R^2$ Wasserstoff ist

d) ein Imidocarbonat VI

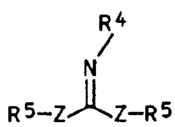

VI

wobei Z für O oder S steht und für Z = O

$R^4$ eine Cyan-, Benzoyl- oder Methansulfonsäuregruppe und $R^5$ eine Phenylgruppe bedeuten oder

für Z = S

$R^4$ eine Cyan- und $R^5$ eine Methylgruppe bedeuten, stufenweise mit dem Amin VIIa oder VIIb

$$R^1\text{-}NH_2 \qquad VIIa, \qquad R^2\text{-}NH_2 \qquad VIIb$$

und dem Diamin III umsetzt und anschliepend hydrolysiert,

oder

e) aus Bromcyan mit dem Amin VIIa bzw. VIIb das N-substituierte Cyanamid VIIIa bzw. VIIIb

$$R^1\text{-}NH\text{-}CN \qquad VIIIa, \qquad R^2\text{-}NH\text{-}CN \qquad VIIIb$$

herstellt und dieses anschließend mit einem mineralsauren Salz des Diamins III zu dem entsprechenden Salz der Verbindung I umsetzt, oder

f) aus Bromcyan und dem Diamin III das N,N'-disubstituierte Cyanamid IX

$$NC\text{-}NH\text{-}(CH_2)_n\text{-}X\text{-}(CH_2)_n\text{-}NH\text{-}CN \qquad IX$$

herstellt und dieses anschliepend mit einem mineralsauren Salz des Amins VIIa bzw. VIIb zu dem Salz der Verbindung I umsetzt,

und die erhaltenen Umsatzprodukte, soweit sie nicht bereits in Form der freien Basen oder der pflanzenverträglichen Salze vorliegen, in die freien Basen oder ihre pflanzenverträglichen Salze überführt oder

aus den freien Basen die Metallkomplexe herstellt.

3. Fungizides Mittel, enthaltend eine fungizid wirksame Menge eines Bis-Guanidins der Formel I,

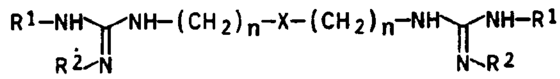

I

in der

R¹ und R² unabhängig voneinander Wasserstoff, $C_3$-$C_{10}$-Alkenyl, $C_3$-$C_{10}$-Alkinyl, $C_1$-$C_8$-Alkoxy-$C_2$-$C_8$-alkyl, $C_3$-$C_{10}$-Halogenalkenyl; oder eine Cycloalkylgruppe mit 5 bis 12 Kohlenstoffatomen im Ring, die bis zu drei der folgenden Substituenten Hydroxyl, $C_1$-$C_{10}$-Alkyl, $C_1$-$C_{10}$-Alkoxy, $C_1$-$C_{10}$-Halogenalkyl und $C_1$-$C_8$-Alkoxy-$C_1$-$C_8$-alkyl tragen kann; oder eine Benzylgruppe, die durch bis zu drei $C_1$-$C_{10}$-Alkyl- oder $C_1$-$C_{10}$-Alkoxy-Gruppen substituiert sein kann, wobei der Substituent noch eine Hydroxyl- oder $C_1$-$C_6$-Alkoxygruppe tragen kann; oder die Benzylgruppe durch eine $C_1$-$C_{10}$-Halogenalkyl-Gruppe substituiert ist, mit der Mapgabe, daß nur einer der beiden Substituenten $R^1$ oder $R^2$ Wasserstoff sein kann; oder

$R^1$ und $R^2$ zusammen mit den Atomen, deren Substituenten sie sind, einen 5- bis 11-gliedrigen heterocyclischen Ring bedeuten, der durch $C_1$-$C_{10}$-Alkyl oder $C_1$-$C_8$-Alkoxy-$C_1$-$C_8$-alkyl ein- bis dreifach substituiert sein kann;

X $CH_2$, O, eine C-C-Einfachbindung, NH, N-($C_1$-$C_{10}$-Alkyl) oder N-Benzyl, wobei der Phenylring des Benzylrestes einfach bis dreifach durch $C_1$-$C_{10}$-Alkyl, $C_1$-$C_{10}$-Alkoxy, $C_1$-$C_{10}$-Halogen-alkyl oder Halogen substituiert sein kann,

n 5 bis 8 bedeuten,

oder dessen pflanzenverträglichen Salzes oder dessen Metallkomplexes und einen flüssigen oder festen Trägerstoff.

4. Verfahren zur Bekämpfung von Pilzen, dadurch gekennzeichnet, daß man eine fungizid wirksame Menge eines Bis-Guanidins der Formel I

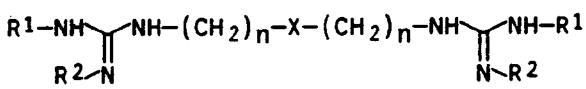

I

in der

$R^1$ und $R^2$ unabhängig voneinander Wasserstoff, $C_3$-$C_{10}$-Alkenyl, $C_3$-$C_{10}$-Alkinyl, $C_1$-$C_8$-Alkoxy-$C_2$-$C_8$-alkyl, $C_3$-$C_{10}$-Halogenalkyl; oder eine Cycloalkylgruppe mit 5 bis 12 Kohlenstoffatomen im Ring, die bis zu drei der folgenden Substituenten Hydroxyl, $C_1$-$C_{10}$-Alkyl, $C_1$-$C_{10}$-Alkoxy, $C_1$-$C_{10}$-Halogenalkyl und $C_1$-$C_8$-Alkoxy-$C_1$-$C_8$-alkyl tragen kann; oder eine Benzylgruppe, die durch bis zu drei $C_1$-$C_{10}$-Alkyl- oder $C_1$-$C_{10}$-Alkoxy-Gruppen substituiert sein kann, wobei der Substituent noch eine Hydroxyl- oder $C_1$-$C_6$-Alkoxygruppe tragen kann; oder die Benzylgruppe durch eine $C_1$-$C_{10}$-Halogenalkyl-Gruppe substituiert ist, mit der Maßgabe, daß nur einer der beiden Substituenten $R^1$ oder $R^2$ Wasserstoff sein kann; oder

$R^1$ und $R^2$ zusammen mit den Atomen, deren Substituenten sie sind, einen 5- bis 11-gliedrigen heterocyclischen Ring bedeuten, der durch $C_1$-$C_{10}$-Alkyl oder $C_1$-$C_8$-Alkoxy-$C_1$-$C_8$-alkyl ein- bis dreifach substituiert sein kann;

X $CH_2$, O, eine C-C-Einfachbindung, NH, N-($C_1$-$C_{10}$-Alkyl) oder N-Benzyl, wobei der Phenylring des Benzylrestes einfach bis dreifach durch $C_1$-$C_{10}$-Alkyl, $C_1$-$C_{10}$-Alkoxy, $C_1$-$C_{10}$-Halogenalkyl oder Halogen substituiert sein kann,

n 5 bis 8 bedeuten,

oder dessen Salzes oder Metallkomplexes auf Pilze, vom Pilzbefall bedrohte Pflanzen, deren Lebensraum, deren Saatgut oder auf Materialien einwirken läßt.

**5.** 1,13-Bis-(3,3-dimethyl-cyclohexyl)-guanidino-7-aza-tridecan-dihydro-iodid.

**6.** 1,17-Bis-cyclohexylguanidino-9-aza-heptadecan-tri-acetat.

**7.** 1,13-Bis-(N,N'-dicyclohexyl)guanidino-7-aza-tridecan-tri-hydrochlorid.

**8.** 1,12-Bis-(2-amino-3,4,5,6-tetrahydro-pyrimidinyl)-dodecan-di-acetat.

## Claims

**1.** A substituted bisguanidine of the formula I

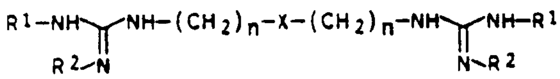

I

where

$R^1$ and $R^2$ are each, independently of one another, hydrogen, $C_3$-$C_{10}$-alkenyl, $C_3$-$C_{10}$-alkynyl, $C_1$-$C_8$-alkoxy-$C_2$-$C_8$-alkyl, $C_3$-$C_{10}$-haloalkenyl; or cycloalkyl which has 5 - 12 carbon atoms in the ring and can carry up to three of the following substituents: hydroxyl, $C_1$-$C_{10}$-alkyl, $C_1$-$C_{10}$-alkoxy, $C_1$-$C_{10}$-haloalkyl and $C_1$-$C_8$-alkoxy-$C_1$-$C_8$-alkyl;
or benzyl which can be substituted up to three times by $C_1$-$C_{10}$-alkyl or $C_1$-$C_{10}$-alkoxy, it being possible for the substituent also to carry a hydroxyl or $C_1$-$C_6$-alkoxy group; or the benzyl is substituted by $C_1$-$C_{10}$-haloalkyl,
with the proviso that only one of $R^1$ and $R^2$ can be hydrogen; or

$R^1$ and $R^2$ are, together with the atoms which they substitute, a 5- to 11-membered heterocyclic ring which can be substituted once to three times by $C_1$-$C_{10}$-alkyl or $C_1$-$C_8$-alkoxy-$C_1$-$C_8$-alkyl;

X is $CH_2$, O, a single bond, NH, N-($C_1$-$C_{10}$-alkyl) or N-benzyl whose phenyl can be substituted once to three times by $C_1$-$C_{10}$-alkyl, $C_1$-$C_{10}$-alkoxy, $C_1$-$C_{10}$-haloalkyl or halogen, and

n is 5 to 8,

and its plant-compatible acid addition salts and its metal complexes.

**2.** A process for preparing a bisguanidine of the formula I

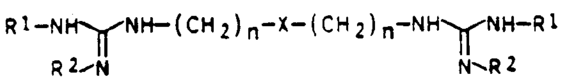

I

where

$R^1$ and $R^2$ are each, independently of one another, hydrogen, $C_3$-$C_{10}$-alkenyl, $C_3$-$C_{10}$-alkynyl, $C_1$-$C_8$-alkoxy-$C_2$-$C_8$-alkyl, $C_3$-$C_{10}$-haloalkenyl; or cycloalkyl which has 5 - 12 carbon atoms in the ring and can carry up to three of the following substituents: hydroxyl, $C_1$-$C_{10}$-alkyl, $C_1$-$C_{10}$-alkoxy, $C_1$-$C_{10}$-haloalkyl and $C_1$-$C_8$-alkoxy-$C_1$-$C_8$-alkyl;
or benzyl which can be substituted up to three times by $C_1$-$C_{10}$-alkyl or $C_1$-$C_{10}$-alkoxy, it being possible for the substituent also to carry a hydroxyl or $C_1$-$C_6$-alkoxy group; or the benzyl is substituted by $C_1$-$C_{10}$-haloalkyl,
with the proviso that only one of $R^1$ and $R^2$ can be hydrogen; or

$R^1$ and $R^2$ are, together with the atoms which they substitute, a 5- to 11-membered heterocyclic ring which can be substituted once to three times by $C_1$-$C_{10}$-alkyl or $C_1$-$C_8$-alkoxy-$C_1$-$C_8$-alkyl;

X is $CH_2$, O, a single bond, NH, N-($C_1$-$C_{10}$-alkyl) or N-benzyl whose phenyl can be substituted once to three times by $C_1$-$C_{10}$-alkyl, $C_1$-$C_{10}$-alkoxy, $C_1$-$C_{10}$-haloalkyl or halogen, and

n is 5 to 8,

which comprises

a) reacting an isothiuronium salt II

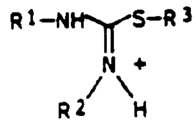 $Y'^-$ II

with a diamine III

$H_2N-(CH_2)_n-X-(CH_2)_n-NH_2$ III

where $R^3$ is benzyl or $C_1$-$C_4$-alkyl, and Y' is an anion, eg. halide, sulfate, methosulfate, $C_1$-$C_4$-alkanesulfonate or p-toluenesulfonate,

or

b) reacting an aminoiminomethanesulfonic acid IV

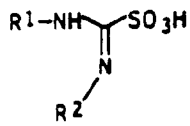 IV

with the diamine III,

or

c) reacting a carbodiimide V

$R^1-N=C=N-R^2$ V

with the diamine III,

or

if one of $R^1$ and $R^2$ is hydrogen

d) reacting an imidocarbonate VI

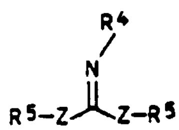 VI

where Z is O or S and, when Z = O, $R^4$ is cyano, benzoyl or methanesulfonyl and $R^5$ is phenyl, or, when Z = S, $R^4$ is cyano and $R^5$ is methyl, stepwise with the amine VIIa or VIIb

$R^1-NH_2$ VIIa, $R^2NH_2$ VIIb

and the diamine III, and subsequently hydrolyzing, or

e) preparing from cyanogen bromide with the amine VIIa or VIIb the N-substituted cyanamide VIIIa or VIIIb

$R^1-NH-CN$ VIIIa, $R^2-NH-CN$ VIIIb

and subsequently reacting the latter with a salt of the diamine III with a mineral acid to give the corresponding salt of the compound I, or

f) preparing from cyanogen bromide and the diamine III the N,N'-disubstituted cyanamide IX

$NC-NH-(CH_2)_n-X-(CH_2)_n-NH-CN$ IX

and subsequently reacting the latter with a salt of the amine VIIa or VIIb with a mineral acid to give the salt of the compound I,

and converting the resulting product if not already in the form of the free base or of the plant-compatible salt into the free base or its plant-compatible salt, or preparing a metal complex from the free base.

3. A fungicide containing an effective amount of a bisguanidine of the formula I

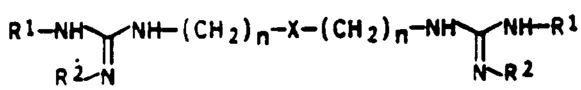 I

where

R¹ and R² are each, independently of one another, hydrogen, $C_3$-$C_{10}$-alkenyl, $C_3$-$C_{10}$-alkynyl, $C_1$-$C_8$-alkoxy-$C_2$-$C_8$-alkyl, $C_3$-$C_{10}$-haloalkenyl; or cycloalkyl which has 5 - 12 carbon atoms in the ring and can carry up to three of the following substituents: hydroxyl, $C_1$-$C_{10}$-alkyl, $C_1$-$C_{10}$-alkoxy, $C_1$-$C_{10}$-haloalkyl and $C_1$-$C_8$-alkoxy-$C_1$-$C_8$-alkyl; or benzyl which can be substituted up to three times by $C_1$-$C_{10}$-alkyl or $C_1$-$C_{10}$-alkoxy, it being possible for the substituent also to carry a hydroxyl or $C_1$-$C_6$-alkoxy group; or the benzyl is substituted by $C_1$-$C_{10}$-haloalkyl, with the proviso that only one of $R^1$ and $R^2$ can be hydrogen; or

R¹ and R² are, together with the atoms which they substitute, a 5- to 11-membered heterocyclic ring which can be substituted once to three times by $C_1$-$C_{10}$-alkyl or $C_1$-$C_8$-alkoxy-$C_1$-$C_8$-alkyl;

X is $CH_2$, O, a single bond, NH, N-($C_1$-$C_{10}$-alkyl) or N-benzyl whose phenyl can be substituted once to three times by $C_1$-$C_{10}$-alkyl, $C_1$-$C_{10}$-alkoxy, $C_1$-$C_{10}$-haloalkyl or halogen, and

n is 5 to 8,

or its plant-compatible salt or its metal complex and a liquid or solid carrier.

4. A process for controlling fungi, which comprises allowing an effective amount of a bisguanidine of the formula I

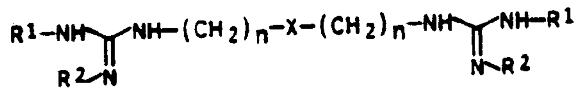

I

where

R¹ and R² are each, independently of one another, hydrogen, $C_3$-$C_{10}$-alkenyl, $C_3$-$C_{10}$-alkynyl, $C_1$-$C_8$-alkoxy-$C_2$-$C_8$-alkyl, $C_3$-$C_{10}$-haloalkyl; or cycloalkyl which has 5 - 12 carbon atoms in the ring and can carry up to three of the following substituents: hydroxyl, $C_1$-$C_{10}$-alkyl, $C_1$-$C_{10}$-alkoxy, $C_1$-$C_{10}$-haloalkyl and $C_1$-$C_8$-alkoxy-$C_1$-$C_8$-alkyl;
or benzyl which can be substituted up to three times by $C_1$-$C_{10}$-alkyl or $C_1$-$C_{10}$-alkoxy, it being possible for the substituent also to carry a hydroxyl or $C_1$-$C_6$-alkoxy group; or the benzyl is substituted by $C_1$-$C_{10}$-haloalkyl,
with the proviso that only one of $R^1$ and $R^2$ can be hydrogen; or

R¹ and R² are, together with the atoms which they substitute, a 5- to 11-membered heterocyclic ring which can be substituted once to three times by $C_l$-$C_{10}$-alkyl or $C_1$-$C_8$-alkoxy-$C_1$-$C_8$-alkyl;

X is $CH_2$, O, a single bond, NH, N-($C_1$-$C_{10}$-alkyl) or N-benzyl whose phenyl can be substituted once to three times by $C_1$-$C_{10}$-alkyl, $C_1$-$C_{10}$-alkoxy, $C_1$-$C_{10}$-haloalkyl or halogen, and

n is 5 to 8,

or its salt or metal complex to act on fungi, plants at risk of fungal attack, their habitat or their seed, or on materials.

5. 1,13-Bis(3,3-dimethylcyclohexylguanidino)-7- azatridecane dihydroiodide.

6. 1,17-Bis cyclohexylguanidino-9-azaheptadecane triacetate.

7. 1,13-Bis(N,N'-dicyclohexylguanidino)-7-azatridecane trihydrochloride.

8. 1,12-Bis(2-amino-3,4,5,6-tetrahydropyrimidyl)-dodecane diacetate.

## Revendications

1. Bis-guanidines substituées de formule générale I

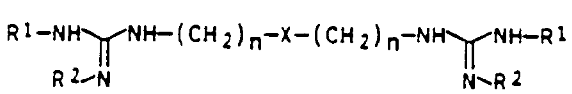

I

dans laquelle

$R^1$ et $R^2$ représentent chacun, indépendamment l'un de l'autre, l'hydrogène, un groupe alcényle en C3-C10, alcynyle en C3-C10, (alcoxy en C1-C8)-alkyle en C2-C8, halogénoalcényle en C3-C10 ; ou un groupe cycloalkyle contenant 5 à 12 atomes de carbone dans le cycle et qui peut porter jusqu'à trois des substituants suivants : les groupes hydroxy, alkyle en C1-C10, alcoxy en C1-C10, halogénoalkyle en C1-C10 et (alcoxy en C1-C8)-alkyle en C1-C8 ;
ou bien un groupe benzyle qui peut porter jusqu'à trois substituants alkyle en C1-C10 ou alcoxy en C1-C10, le substituant pouvant encore porter un groupe hydroxy ou alcoxy en C1-C6 ; ou bien le groupe benzyle est substitué par un groupe halogénoalkyle en C1-C10, sous réserve qu'un seul des symboles R1 ou $R^2$ peut représenter l'hydrogène ; ou bien

$R^1$ et $R^2$ forment ensemble et avec les atomes dont ils sont substituants, un noyau hétérocyclique de 5 à 11 chaînons qui peut être mono- à tri-substitué par des groupes alkyle en C1-C10 ou (alcoxy en C1-C8)-alkyle en C1-C8 ;

X représente $CH_2$ , O, une liaison C-C simple, NH, N-(alkyle en C1-C10) ou N-benzyle, le noyau phényle du radical benzyle pouvant porter un à trois substituants alkyle en C1-C10, alcoxy en C1-C10, halogénoalkyle en C1-C10 ou halogéno, et

n est un nombre allant de 5 à 8,

ainsi que leurs sels formés par addition avec des acides et complexes métalliques tolérés par les végétaux.

2. Procédé de préparation des bis-guanidines de formule I

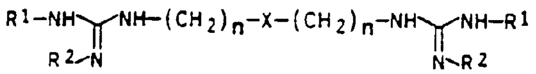

I

dans laquelle

$R^1$ et $R^2$ représentent chacun, indépendamment l'un de l'autre, l'hydrogène, un groupe alcényle en C3-C10, alcynyle en C3-C10, (alcoxy en C1-C8)-alkyle en C2-C8, halogenoalcényle en C3-C10 ; ou un groupe cycloalkyle contenant 5 à 12 atomes de carbone dans le cycle et qui peut porter jusqu'à trois des substituants suivants :
les groupes hydroxy, alkyle en C1-C10, alcoxy en C1-C10, halogénoalkyle en C1-C10 et (alcoxy en C1-C8)-alkyle en C1-C8 ;
ou bien un groupe benzyle qui peut porter jusqu'à trois substituants alkyle en C1-C10 ou alcoxy en C1-C10, le substituant pouvant encore porter un groupe hydroxy ou alcoxy en C1-C6 ; ou bien le groupe benzyle est substitué par un groupe halogénoalkyle en C1-C10, sous réserve qu'un seul des symboles R1 et $R^2$ peut représenter l'hydrogène ; ou bien

$R^1$ et $R^2$ forment ensemble et avec les atomes dont ils sont substituants, un noyau hétérocyclique de 5 à 11 chaînons qui peut être mono- à tri-substitué par des groupes alkyle en C1-C10 ou (alcoxy en C1-C8)-alkyle en C1-C8 ;

X représente $CH_2$ , O, une liaison C-C simple, NH, N-(alkyle en C1-C10) ou N-benzyle, le noyau phényle du radical benzyle pouvant être mono- à tri-substitué par des groupes alkyle en C1-C10, alcoxy en C1-C10, halogénoalkyle en C1-C10 ou des halogènes, et

n est un nombre allant de 5 à 8,

caractérisé en ce que

a) on fait réagir un sel d'isothiuronium II

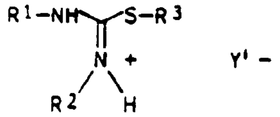

II

avec une diamine III

$$H_2N\text{-}(CH_2)_n\text{-}X\text{-}(CH_2)_n\text{-}NH_2 \qquad III$$

$R^3$ représentant un groupe benzyle ou alkyle en C1-C4 et Y' un anion, par exemple un anion halogénure, sulfate, méthylsulfate, alkylsulfonate en C1-C4 ou p-tolylsulfonate, ou bien

b) on fait réagir un acide aminoiminométhane-sulfonique IV

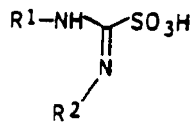

IV

avec une diamine III
ou bien
c) on fait réagir un carbodiimide V

$R^1$-N=C=N-$R^2$ V

avec la diamine III,
ou bien
lorsqu'un des symboles $R^1$ ou $R^2$ représente l'hydrogène,
d) on fait réagir un imidocarbonate VI

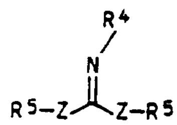

VI

pour lequel Z représente O ou S, et, lorsque Z = O, $R^4$ représente un groupe cyano, benzoyle ou méthane-sulfonyle et $R^5$ un groupe phényle, ou bien lorsque Z = S, $R^4$ représente un groupe cyano et $R^5$ un groupe méthyle, successivement, avec l'amine VIIa ou VIIb

$R^1$-$NH_2$ VIIa, $R^2$-$NH_2$ VIIb

et la diamine III, puis on hydrolyse,
ou bien
e) on prépare, à partir du bromocyanogène et de l'amine VIIa ou VIIb, le cyanamide substitué à l'azote VIIIa ou VIIIb

$R^1$-NH-CN VIIIa, $R^2$-NH-CN VIIIb

qu'on fait ensuite réagir avec un sel d'acide minéral de la diamine III, ce qui donne le sel correspondant du composé I, ou bien
f) on prépare à partir du bromocyanogène et de la diamine III, le cyanamide N, N' disubstitué IX

NC-NH-$(CH_2)_n$-X-$(CH_2)_n$-NH-CN IX

qu'on fait ensuite réagir avec un sel d'acide minéral de l'amine VIIIa ou VIIIb, ce qui donne le sel du composé I,

et, lorsqu'ils ne sont pas déjà à l'état de bases libres ou de sels tolérés par les végétaux, on convertit les produits obtenus en les bases libres ou leurs sels tolérés par les végétaux ou bien on prépare les complexes métalliques à partir des bases libres.

**3.** Produit fongicide contenant une quantité fongicide efficace d'une bis-guanidine de formule I

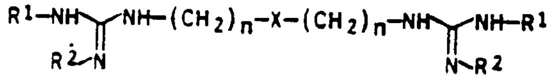

I

dans laquelle

$R^1$ et $R^2$ représentent chacun, indépendamment l'un de l'autre, l'hydrogène, un groupe alcényle en C3-C10, alcynyle en C3-C10, (alcoxy en C1-C8)-alkyle en C2-C8, halogénoalcényle en C3-C10 ; ou un groupe cycloalkyle contenant 5 à 12 atomes de carbone dans le cycle et qui peut porter jusqu'à trois des substituants suivants : les groupes hydroxy, alkyle en C1-C10, alcoxy en C1-C10, halogénoalkyle en C1-C10 et (alcoxy en C1-C8)-alkyle en C1-C8 ;
ou un groupe benzyle qui peut porter jusqu'à trois substituants alkyle en C1-C10 ou alcoxy en C1-C10, le substituant pouvant encore porter un groupe hydroxy ou alcoxy en C1-C6 ; ou bien le groupe benzyle est substitué par un groupe halogénoalkyle en C1-C10, sous réserve qu'un seul des symboles $R^1$ et $R^2$ peut représenter l'hydrogène ; ou bien

$R^1$ et $R^2$ forment ensemble et avec les atomes dont ils sont substituants un noyau hétérocyclique de 5 à 11 chaînons qui peut porter un à trois substituants alkyle en C1-C10 ou (alcoxy en C1-C8)-alkyle en C1-C8 ;

X représente $CH_2$ , O, une liaison C-C simple, NH, N-(alkyle en C1-C10) ou N-benzyle, le noyau phényle du radical benzyle pouvant porter un à trois substituants alkyle en C1-C10, alcoxy en C1-C10, halogénoalkyle en C1-C10, ou halogéno,

n est un nombre allant de 5 à 8,

ou d'un de ses sels ou complexe métallique toléré par les végétaux, et un véhicule liquide ou solide.

**4.** Procédé pour combattre les mycètes, caractérisé en ce que l'on fait agir sur les mycètes, les végétaux menacés par une attaque par les mycètes, leur habitat, leurs semences, ou sur des matériaux, une quantité fongicide efficace d'une bis-guanidine de formule I

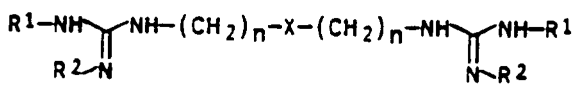

I

dans laquelle

$R^1$ et $R^2$ représentent chacun, indépendamment l'un de l'autre, l'hydrogène, un groupe alcényle en C3- C10, alcynyle en C3-C10, (alcoxy en C1-C8)-alkyle en C2-C8, halogénoalcényle en C3-C10 ; ou un groupe cycloalkyle contenant 5 à 12 atomes de carbone dans le cycle et qui peut porter jusqu'à trois des substituants suivants : les groupes hydroxy, alkyle en C1-C10, alcoxy en C1-C10, halogénoalkyle en C1-C10 et (alcoxy en C1-C8)-alkyle en C1-C8 ;
ou un groupe benzyle qui peut porter jusqu'à trois substituants alkyle en C1-C10 ou alcoxy en C1-C10, le substituant pouvant encore porter un groupe hydroxy ou alcoxy en C1-C6 ; ou bien le groupe benzyle est substitué par un groupe halogénoalkyle en C1-C10, sous réserve qu'un seul des symboles $R^1$ et $R^2$ peut représenter l'hydrogène ; ou bien

$R^1$ et $R^2$ forment ensemble et avec les atomes dont ils sont substituants un noyau hétérocyclique de 5 à 11 chaînons qui peut porter un à trois substituants alkyle en C1-C10 ou (alcoxy en C1-C8)-alkyle en C1-C8 ;

X représente $CH_2$ , O, une liaison C-C simple, NH, N-(alkyle en C1-C10) ou N-benzyle, le noyau phényle du radical benzyle pouvant porter un à trois substituants alkyle en C1-C10, alcoxy en C1-C10, halogénoalkyle en C1-C10, ou halogéno, et

n est un nombre allant de 5 à 8,

ou de l'un de ses sels ou complexe métallique.

**5.** Le diiodhydrate du 1,13-bis-(3,3-diméthylcyclohexyl)guanidino-7-aza-tridécane.

**6.** Le triacétate du 1,17-bis-cyclohexylguanidino-9-aza-heptadécane.

**7.** Le trichlorhydrate du 1,13-bis-(N-N'-dicyclohexyl)-guanidino-7-aza-tridécane.

**8.** Le diacétate du 1,12-bis-(2-amino-3,4,5,6-tétrahydro-pyrimidinyl)-dodécane.